# EUROPÄISCHE PATENTANMELDUNG

(11) **EP 0 885 617 A2**
(43) Veröffentlichungstag der Anmeldung: **23.12.1998**
(21) Anmeldenummer: 98110667.7
(22) Anmeldetag: 10.06.1998
(51) Int. Cl.: A61L 27/00, A61L 25/00

(54) **Implantatmaterial mit einer Träger-Wirkstoff-Kombination**

(30) Priorität: 21.06.1997 DE 19726412
(71) Anmelder: MERCK PATENT GmbH, 64293 Darmstadt (DE)
(72) Erfinder: Nies, Berthold, Dr., 64407 Fränkisch-Crumbach (DE)

(57) **Zusammenfassung**

Die vorliegende Erfindung betrifft poröse Implantatmaterialien, welche mit einer Träger-Wirkstoff-Kombination beladen sind, wobei als Träger-Komponente oligomere Ester aus mehrwertigen Alkoholen und α-Hydroxysäuren verwendet werden.

## Beschreibung

Die Erfindung betrifft poröse Implantatmaterialien, welche mit einer Träger-Wirkstoff-Kombination beladen sind, wobei als Träger-Komponente oligomere Ester aus mehrwertigen Alkoholen und α-Hydroxysäuren verwendet werden.

Unter porösen Implantatmaterialien sind beispielsweise Knochenersatzmaterialien zu verstehen, die als Implantate für den Ersatz oder die Rekonstitution von Knocherstrukturen aufgrund von Defekten nach krankheits- oder unfallbedingten operativen Eingriffen dienen. Beispielhaft zu nennen sind Implantatformkörper wie Knochenprothesen verschiedenster Art, Knochenverbindungselemente etwa in Form von Markraumnägeln, Knochenschrauben und Osteosyntheseplatten, Implantatmaterialien zur Auffüllung von Spongiosa-Knochendefekten oder von Zahnextraktionshöhlen sowie zur plastisch-chirurgischen Behandlung von Konturdefekten im Kiefer-Gesichtsbereich.

Weiterhin fallen unter diesen Begriff oberflächenstrukturierte Implantate, wie z.B. Zahnimplantate oder Metallprothesen für den Ersatz von Gelenken. Ferner sind dar-unter auch Vliese, Membranen, Gewebe, Laminate und Ähnliches für Wundabdeckungen, Hautersatz, Gefäßprothesen oder auch Bandersatz zu verstehen.

Für den Einheilungsprozess werden solche Implantatmaterialien als besonders günstig angesehen, die eine hohe Bioaktivität aufweisen, nämlich dahingehend, dass sie im Organismus angenommen und in ihm integriert werden. Im Falle von Knochenersatzmaterial bedeutet dies, dass es bald mit körpereigenem Gewebe, insbesondere mit dem Knochen, fest und dauerhaft verwachsen soll.

In der DE 41 21 043 ist ein Knochenersatzmaterial beschrieben, das in einer porösen Matrix ein oder mehrere Polypeptide mit der biologischen Wirkung von Fibroblasten-Wachstumsfaktoren enthält. Dort werden zwar gute Ergebnisse bei der Stimulation des Knocheneinwuchses in die poröse Struktur des Implantats erzielt, jedoch gibt es auch einige noch ungelöste Probleme. Einerseits kann die Stabilität der gelöst aufgebrachten peptidischen Wachstumsfaktoren Probleme bereiten und andererseits kann die biologische Wirkung durch die unkontrollierte Freisetzung nicht optimal und reprodusierbar eingestellt werden. Diese Probleme der Stabilität und der unkontrollierten Freisetzung sind auch bei anderen Wirkstoffen, wie zum Beispiel Antibiotika, Cytostatika oder anderen wachstumsfördernde Substanzen, mit welchen das Implantatmaterial beladen ist, zu finden.

Es bestand daher die Aufgabe, eine, den oder die Wirkstoff(e) enthaltende Kombination zu finden, die leicht auf das poröse Implantatmaterial aufgebracht werden kann, die auch empfindliche Wirkstoffe schützt, damit ihre Wirksamkeit erhalten bleibt, und wobei die Abgabe aus dem porösen Implantat in einer Weise erfolgt, die eine Reproduzierbarkeit der biologisch/pharmakologischen Wirkung ergibt.

Es wurde nun gefunden, dass ein System, bestehend aus einer Trägerkomponente, die im wesentlichen aus einem oligomeren Ester aus mehrwertigen Alkoholen und α-Hydroxysäuren besteht, und einer oder mehreren Wirkstoffkomponenten, diese Probleme hervorragend bewältigen kann.

Gegenstand der Erfindung ist daher ein poröses Implantatmaterial, welches mit einer Träger-Wirkstoff-Kombination beladen ist, wobei als Träger oligomere Ester aus mehrwertigen Alkoholen und α-Hydoxysäuren verwendet werden.

Gegenstand der Erfindung ist auch ein Verfahren zur Herstellung eines Implantatmaterials, welches mit einer Träger-Wirkstoff-Kombination beladen ist, das dadurch gekennzeichnet ist, dass man die oligomeren Ester in einem organischen Lösungsmittel auflöst und darin den oder die Wirkstoff(e) einbringt, und dass man anschließend diese Mischung auf das poröse Implantatmaterial aufbringt, das Lösungsmittel entfernt und gegebenenfalls das Implantatmaterial zuletzt steril verpackt.

Als sogenannte Trägerkomponente werden also oligomere Ester aus mehrwertigen Alkoholen und α-Hydoxysäuren (im Folgenden auch kurz AHA genannt) verwendet. Grundsätzlich sind solche Polymere bzw. Oligomere bekannt. Beispielsweise ist bekannt, dass hochpolymere Ester ausgewählter niederer Hydroxysäuren, und zwar inbesondere der Milchsäure, hohe Körpervertäglichkeit besitzen und in der Operationstechnik beispielsweise als körperverträgliches und körperresorbierbares Fadenmaterial eingesetzt werden, das im Laufe von Wochen bzw. Monaten abgebaut und aus dem Körper ausgeschwemmt wird.

Oligomere Ester insbesondere der Milchsäure und/oder der Gykolsäure, die einen mittleren Oligomerisierungsgrad von bis zu 100 aufweisen, sind aus der DE 36 20 685 bekannt. Dort wird die Verwendung dieser Ester als resorbierbarer Träger und/oder Filmbildner in Mitteln zu Abdeckung menschlicher oder tierischer Haut beschrieben.

Die in der Erfindung verwendeten Oligomeren zeichnen sich durch einen mittleren Oligomerisierungsgrad der gewählten Säure bis zu etwa 30 und vorzugsweise bis zu etwa 10 aus.

Die Herstellung solcher Oligomeren aus Hydroxycarbonsäuren ist bekannt und kann im Prinzip unmittelbar durch Polykondensation der Hydroxycarbonsäuren bzw. Hydroxycarbonsäuregemische durchgeführt werden, jedoch ist es zur gezielten Einstellung des Oligomerisierungsgrades sinnvoll, in bekannter Weise Coreaktanten zur Regulierung des Oligomerisierungsgrades zuzufügen, wobei hier in erster Linie einoder mehrwertige Alkohole oder organische Säuren in Betracht kommen können. Die beiden Ester bildenden Gruppen der Monomeren bzw. der Oligomeren, d.h. die Hydroxylgruppe einerseits und die Carboxylgruppe andererseits, bieten sich ja all-gemein als reaktive Stellen dafür an.

Insbesondere kommen in bevorzugten Ausführungsformen Alkohole mit bis zu 4, insbesondere mit bis zu 3 Hydroxylgruppen in Betracht. Im zuletzt genannten Fall ist es insbesondere das Glycerin, welches durch Umsetzung mit den AHA-Oligomeren zu ausserordentlich vielgestaltigen Produkten führt. Beispiele für weitere, besonders bevorzugte Alkohole sind: Ethylenglycol, 1,2- oder 1,3-Propylenglycol, Butylenglycol, Trimethylolpropan, niedermolekulare Polyethylengkykole, Polypropylenglykol, 1,5-Pentandiol oder auch höherwertige Alkohole, Zucker oder Zuckeralkhole.

Auf dem Gebiet der Mitverwendung von Carborsäuren können einerseits physiologisch verträgliche Carbonsäuren, hier insbesondere Monocarbonsäuren interessant sein, aber auch poylfunktionelle Carbonsäuren, beispielsweise Di- oder Tricarbonsäuren.

Die Herstellung der oligomeren Hydroxycarbonsäuren bzw. ihrer Derivate erfolgt in an sich bekannter Weise. Selbstverständlich können in allen Fällen -d.h. sowohl bei den α-Hydroxcarbonsäuren als auch bei den Coreaktanten - nicht nur die jeweils freien reaktiven Komponenten der genannten Art, sondern auch solche Derivate eingesetzt werden, die in an sich bekannter Weise unter den Bedingungen der Veresterung bzw. Umesterung die gewünschten Polyester-Oligomeren bilden. Geeignet sind also beispielsweise die Ester der Hydroxysäuren, ferner wird man auch die leicht handhabbaren Dimerisationsprodukte z.B. der Milchsäure und/oder Glykolsäure, d.h. das Lactid und/oder das Glykolid, einsetzen.

Die Polykondensationsreaktion wird dabei üblicherweise durch Erhitzen der Ausgangsmaterialien auf eine Temperatur oberhalb des Schmelzpunktes, vorzugsweise in Gegenwart eines Katalysators, insbesondere eines Veresterungskatalystors, unter wasserfreien Bedingungen in einer Inertgasatmosphäre durchgeführt. Menge und Art des verwendeten Katalysators bestimmen die Verfahrenstemperatur und die Dauer der Umsetzung. Da der Umsatz der Reaktionen normalerweise nahezu 100 % beträgt, lässt sich durch die Zugabe der Reaktionsteilnehmer die Zusammensetzung der Endprodukte gut steuern. Dem Fachmann sind solche Polykondensationsreaktionen wohl bekannt. Eine detaillierte Aufzählung der verschiedensten Reaktionsbedingungen ist daher an dieser Stelle nicht notwendig.

Als α-Hydroxysäuren kommen vorzugsweise die im Folgenden aufgeführten Verbindungen zur Verwendung: Glykolsäure und Milchsäure, die beide im Stoffwechsel des lebenden Organismus auftreten und vom Körper verarbeitet bzw. ausgeschieden werden können, wobei die Milchsäure dabei in Form ihres Racemats oder auch in Form ihrer optischen Antipoden oder als beliebige Gemische der optischen Antipoden Verwendung findet; α-Hydroxybuttersäure, α-Hydroxyvaleriansäure, Trimethylencarbonat, ε-Caprolacton oder auch Dioxanon.

Ganz besonders bevorzugt werden dabei Glykolsäure und/oder Milchsäure bzw. ihre Dimerisationsprodukte oder auch Trimethylencarbonat oder Caprolacton eingesetzt.

Erfindungsgemäß ist es möglich, sowohl homooligomere beziehungsweise heterooligomere Ester zu verwenden. Bei sogenannte homooligomeren Estern bedeutet dies, dass nur eine einzige α-Hydroxysäure oder eines ihrer reaktionsfähigen Derivate, z.B. allein die Milchsäure, zur Herstellung der oligomeren Ester eingesetzt wird. Oder bei den Heterooligomeren bedeutet dies, dass bei der Polykondensationsreaktion zur Herstellung der oligomeren Produkte Mischungen unterschiedlicher α-Hydroxysäuren eingesetzt werden. Vorzugsweise tritt auch Glycolsäure, Trimethylencarbonat oder auch Caprolacton als mengenmäßig deutlich kleinererTeil in Cooligomeren mit Milchsäure bzw. anderen α-Hydroxysäuren auf.

Besonders bevorzugte Oligomere werden beispielsweise aus folgenden Monomeren synthetisiert: Glycerin, Lactid und Glycolid; Ethylenglycol, Milchsäure und Glycolsäure; Propylenglycol, Lactid und Glycolid; Glycerin und Lactid; Glycerin, Glycolsäure und α-Hydroxy butyrsäure; Ethylenglycol und α-Hydroxyvaleriansäure; Glycerin und Milchsäureethylester; Glycerin, Trimethlyencarbonat und Glycolsäure; Ethylenglycol und Lactid; Ethylenglycol, Glycolsäure und Milchsäureethylester; Glycerin, Glycolsäure und ε-Caprolacton.

Die Aufzählung der vorangegangenen Verbindungen stellt nur eine kleine Auswahl an besonders bevorzugten Produkten dar, sie soll keinesfalls limitierenden Charakter haben.

Als Wirkstoffe können alle pharmazeutischen Wirkstoffe in Betracht kommen, die von ihrem Wirkungsprofil her in Knochenzementen, in Knochenersatzwerkstoffen, Implantationsmaterialien sowie in implantierbaren Pharmakadepots sinnvoll sind. Als Wirkstoffe kommen somit vorzugsweise Cytostatika wie Methotrexat, Cisplatin, Cyclophosphamid, Fluoruracil, Doxorubicin etc., Antibiotika wie Gentamicin, Clindamycin, Vancomycin, Teicoplanin etc., weiterhin Antiseptika sowie wachstumsfördernde Substanzen in Betracht.

Als wachstumsfördernde Substanzen sollen hier beispielsweise die Fibroblasten-Wachstumsfaktoren (Fibroblast Growth Factors, FGF) erwähnt werden, die zur Klasse der körpereigenen Peptid-Wachstumsfaktoren gehören. FGFs sind bekannt als wirksame gefäßbilder de Faktoren, die u.a. für die Neovaskularisation bei der Wundheilung verantwortlich sind, und auch als Knochenwachstums-fördernde Substanz. Nähere Details zu FGFs einschließlich ihrer Abwandlungsprodukte, zu ihrer Isolierung bzw. Herstellung, ihrer Struktur, ihrer biologischen Aktivitäten und deren Mechanismen sowie zu entsprechenden medizinischen Anwendungen können der inzwischen umfangreichen Fachliteratur entnommen werden. Eine umfassende Übersicht bietet beispielsweise A. Baird and P. Böhlen, Fibroblast Growth Factors, in: Peptide Growth Factors and their Receptors I (editors: M.B. Sporn and A.B. Roberts) Springer Verlag Berlin, Heidelberg, New York 1990.

Die oligomeren Ester sind weitgehend unreaktiv bei normalen Umgebungsbedingungen, sofern sie frei von Feuchtigkeit gehalten werden. Aus diesem Grund sind sie kompatibel mit sehr vielen interessanten Wirkstoffen. Wirkstoffe, die in diese oligomeren Ester eingearbeitet werden, bleiben so über einen langen Zeitraum stabil. Bei der direkten Aufbringung von zum Beispiel Proteinen auf Implantatoberflächen (z.B. Endobon) wird das zuvor gefriergetrocknete Protein in wäßriger Lösung rekonstituiert. Bereits in diesem gelösten Zustand tritt häufig schon eine langsame Inaktivierung ein. Bei der Auftragung auf das Implantat kommt das Protein mit der Trägeroberfläche in Kontakt und kann auf der Oberfläche denaturiert oder irreversibel gebunden werden. Zudem kann, wie im Falle von Endobon, das Implantat lösliche Komponenten abgeben, die beispielsweise den pH-Wert der Beladungslösung stark verändern und dadurch zur Wirkstoffinaktivierung beitragen.

Im erfindungsgemäßen Fall wird der Wirkstoff nicht gelöst, sondern vorzugsweise als mikronisiertes Lyophilisat im oligomeren Ester suspendiert und als mehr oder weniger dicke Schicht auf die Trägeroberfläche aufgebracht. Der Wirkstoff wird also weder gelöst, noch kommt er in gelöster Form mit dem Träger in Verbindung. Erst nach Implantation oder sonstiger Einbringung in den Körper kommt der Wirkstoff mit einer wäßriger Lösung in Kontakt, und die Freigabe geschieht dann sowohl durch langsame Auflösung des hydrolysierbaren oligomeren Esters als auch durch Diffusion von Wasser in die Ester-Matrix, Auflösung des Wirkstoffs in der Matrix und anschließende Diffusion des gelösten Wirkstoffs durch die Matrix. Dieser Vorgang ist durch die abgestimmte Auswahl von oligomeren Ester, Wirkstoffpräparation, Schichtdicke und gegebenenfalls Zusätze, über die man die Freisetzung des Wirkstoffs steuern kann, in weiten Grenzen einstellbar. Solche Zusätze sind vorzugsweise bekannte Substanzen, die die Wasseraufnahme in die oligomeren Ester erhöhen und dadurch die Freisetzung des Wirkstoffs erhöhen. Solche die Freisetzung fördernde Effekte sind beispielsweise von Aminosäuren, Zuckern, bestimmten organischen Lösungsmitteln und auch leicht wasserlöslichen körperverträglichen Stoffen bekannt (DE 44 33 201). Insbesondere im Vergleich zur direkten Beladung mit Wirkstofflösung ergibt sich dadurch eine kontrollierbare und gegen äußere Einflüsse wesentlich unempfindlichere Abgabe des Wirkstoffs.

Die Herstellung der Träger-Wirkstoff-Kombination erfolgt folgendermaßen. Die oligomeren Ester werden in einfachen wasserfreien, organischen Lösungsmitteln aufgelöst. Als Lösungsmittel kommen beispielsweise Aceton, N-Methylpyrrolidon, 2-Pyrrolidon, Ethylacetat, Dioxan oder Tetrahydrofuran in Frage. Der oder die Wirkstoff(e) werden, vorzugsweise als mikronisiertes Lyophilisat, in diesen Lösungen suspendiert oder gelöst. Diese Lösung oder Suspension wird anschließend auf das poröse Implantatmaterial aufgebracht. Anschließend wird das Lösungsmittel entfernt und vorzugsweise verpackt und sterilisiert, beispielweise durch Gammabestrahlung.

Die Art der Beladung hängt unter anderem vom Implantationsmaterial und dem Wirkstoff ab. Die Beladungshöhe kann sehr unterschiedlich sein und hängt sehr stark von der spezifischen Aktivität des Wirkstoffs ab.

Für die Beladungshöhe können folgende beispielhaft genannten Werte als geeignete Richtwerte angegeben werden. Vorzugsweise beträgt die Beladung mit Antibiotika 5 % bis 80 %, insbesondere bevorzugt 10 % bis 60 % (jeweils bezogen auf das Oligomer). Bei den Wachstumsfaktoren (hochwirksame Peptide, Proteine) beträgt die Beladung 0.01 µg/ml bis 250 mg/ml, vorzugsweise 0.1 µg/ml bis 100 mg/ml.

Im Falle von Implantaten, wie Hüft- oder Knieendoprothesen, kann die Suspension vorteilhaft direkt über ein Dosiergerät auf die Metalloberfläche gedruckt werden. Dies kann vorzugsweise über einen programmgesteuerten Roboter geschehen. Andererseits sind natürlich auch Tauchverfahren und Aufsprühtechniken möglich.

Bei porösen Granulaten, wie zum Beispiel Endobon-Granulat, sind die üblichen Techniken aus der pharmazeutischen Technologie für viele Matrix-Wirkstoffkombinationen anwendbar, wie zum Beispiel das Besprühen im Dragierkessel.

Eine andere Möglichkeit besteht auch darin, die einzelnen Komponenten steril zu verpacken und in einem gebrauchsfertigen Set zur Verfügung zu stellen. Somit kann das erfindungsgemäß beladene Implantat direkt vor der Anwendung hergestellt werden.

Somit ist auch Gegenstand der Erfindung ein Implantatmaterial nach einem der Ansprüche 1 bis 6, das dadurch gekennzeichnet ist, dass es in Form eines gebrauchsfertigen Sets aus zwei oder mehr getrennten Komponenten vorliegt, dessen eine Komponente das poröse Implantatmaterial und eine andere Komponente eine Lösung derTräger-Wirkstoff-Kombination beinhaltet, oder gegebenenfalls auch die einzelnen Komponenten enthält, aus welchen die Träger-Wirkstoff-Kombination hergestellt werden kann.

Eine derartige Ausführungsform ist besonders zweckmäßig, um mögliche Stabilitätsprobleme, die bei einer Langzeitlagerung von bereitsfertig konfektionierten erfindungsgemäßen Implantatmaterialien auftreten könnten, wirksam zu begegnen. Die Anwendung der erfindungsgemäßen Implantatmaterialien erfolgt in der Weise, dass man kurz, vor oder während des chirurgischen Eingriffs oder der entsprechenden Behandlung das poröse Implantatmaterial mit der Träger-Wirkstoff-Kombination in der beschriebenen Weise belädt. Dabei können die Trägerlösung und der oder die Wirkstoffe entweder schon gemischt oder auch separat steril verpackt vorliegen. Falls sie separat verpackt sind, müssen diese selbstverständlich vor der Aufbringung auf das Implantatmaterial zuerst wie beschrieben gemischt werden. Die Einzelkomponenten werden dabei vorzugsweise getrennt steril hergestellt und verpackt und bei Bedarf zum gebrauchsfertigen Implantatmaterial unter aseptischen Bedingungen kombiniert.

Für die erfindungsgemaßen Implantatmaterialien können grundsätzlich alle bekannten und üblichen Implantatwerkstoffe verwendet werden, sofern diese eine poröse Matrix oder Oberfläche aufweisen, oder ein oberflächenstrukturiertes Material dar-stellen. Implantatwerkstoffe können in die Klassen mineralische, insbesondere keramische Werkstoffe, physiologisch akzeptable metallische Werkstoffe, physiologisch akzeptable Polymerwerkstoffe und Verbundwerkstoffe aus zwei oder mehr Materialien der genannten Art eingeteilt werden. Diese Werkstoffe können als Ganzes eine poröse Matrix bilden, etwa in Form von porösen Implantatformkörpern oder es können nur bestimmte Anteile des Werkstoffs als poröses Material vorliegen bzw. bestimmte Bereiche eines Implantatformkörpers eine poröse Matrix darstellen. Die letzten beiden Fälle können beispielsweise in der Form realisiert sein, dass ein Verbundwerkstoff oder ein Knochenzement eine poröse Komponente enthält bzw. ein Implantat mit einer porösen Oberflächenbeschichtung oder einer entsprechend aufgerauhten Oberfläche versehen ist.

Als mineralische Werkstoffe kommen beispielsweise Materialien in Frage, die auf Calcium-haltigen Materialien basieren, wie insbesondere Calciumcarbonat, Calciumphosphate und von diesen Verbindungen abgeleitete Systeme. Aus der Gruppe der Calciumphosphate sind als bevorzugt Hydroxylapatit, Tricalciumphosphat und Tetracalciumphosphat zu nennen.

Implantatwerkstoffe auf mineralischer Basis gewährleisten jedoch meist nur dann eine hohe mechanische Stabilität, wenn sie als Keramiken, d.h. also in Form von bei ausreichend hohen Temperaturen gesinterten Materialien bzw. Werkstücken eingesetzt werden.

Näheres zu Knochenkeramiken und besonders günstige Verfahren zu ihrer Herstellung kann beispielsweise den Patentdokumenten DE 37 27 606, DE 39 03 695, DE 41 00 897 und DE 40 28 683 entnommen werden. Der große Vorteil der vorliegenden Erfindung liegt darin, dass die Träger-Wirkstoff-Kombination weitgehend unabhängig vom verwendeten Material, mit den verschiedensten Materialien angewendet werden kann. Die Implantatwerkstoffe müssen nur so geartet oder gestaltet sein, dass sie, wie schon erwähnt, eine poröse Matrix zur Aufnahme der Träger-Wirkstoff-Kombination und Wiederabgabe an den Organismus aufweisen, zweckmäßigerweise zumindest vornehmlich im Kontaktbereich mit dem Körpergewebe. Diese Voraussetzungen erfüllen beispielweise auch Implantate aus metallischen Werkstoffen, die in sich porös sind oder eine poröse Oberflächenbeschichtung, oder die eine porös stukturierte oder zumindest aufgerauhte Oberfläche aufweisen. Gleiches gilt für Implantate aus polymeren Werkstoffen, anderen Keramikmaterialien oder aus Verbundwerkstoffen.

Als Beispiele für solche oberflächenstrukturierten Implantate seien Zahnimplantate oder auch Metallprothesen für Gelenkersatz genannt. Als metallischer Werkstoff wird hauptsächlich Titan oder eine Titanlegierung eingesetzt.

Als weitere geeignete, beispielhaft genannte, Implantatmaterialien seien hier nur noch Materialien erwähnt, die für Wundabdeckungen, als Hautersatz, Gefäßprothesen oder auch Bandersatz eingesetzt werden, wie Vliese, Membranen, Gewebe oder Laminate.

Grundsätzlich können die erfindungsgemäßen Implantatmaterialien nicht nur als Implantatformkörper vorliegen, sonder auch in Pulver- oder Granulatform, je nachdem wie es der Einsatzort und der Anwendungszweck erfordert.

Die vorangegangene Aufzählung an möglichen porösen Implantatmaterialien stellt nur eine Kleine Auswahl an besonders bevorzugten Produkten dar, sie soll keinesfalls limitierenden Charakter haben.

Mit der erfindungsgemäßen Beladung poröser Implantatmaterialien mit der hier beschriebenen Träger-Wirkstoff-Kombination ist es also nun gelungen, ein System herzustellen, welches die empfindlichen Wirkstoffe schützt und stabilisiert und aus welchem ferner der Wirkstoff langsam, über einen längeren Zeitraum hin, kontrolliert abgegeben wird. Somit ist auch eine Reproduzierbarkeit der biologisch/pharmakologischen Wirkung gewährleistet.

Ohne weitere Ausführungen wird davon ausgegangen, dass ein Fachmann die obige Beschreibung in weitestem Umfang nutzen kann. Die bevorzugten Ausführungsformen sind deswegen lediglich als beschreibende, keineswegs als in irgendeine Weise limitierende Offenbarung aufzufassen.

Die vollständige Offenbarung aller vor- und nachstehend aufgeführten Anmeldungen, Patente und Veröffentlichungen sind durch Bezugnahme in diese Anmeldung eingeführt.

Die folgenden Beispiele sollen die vorliegende Erfindung verdeutlichen.

### Beispiel 1

100.0 g Glycerooligolactid der Zusammensetzung 1 : 26 (Teile Glycerin : Teile Milchsäure) werden in 250 ml Aceton gelöst. Zu dieser Lösung werden 15.3 g mikronisiertes Gentamicinsulfat (entsprechend 10 g Aktivität) gegeben und es wird intensiv gemischt. Die so erhaltene Suspension wird auf einen Hüftprothesenschaft aus Titanlegierung aufgebracht, so dass die Beladung je cm² Oberfläche 2.5 mg an Gentamicinaktivität beträgt. Anschließend wird bei einer Temperatur von 30 °C das Aceton entfernt, der Prothesenschaft verpackt und in der Verpackung durch Gammabestsrahlung sterilisiert. Das Produkt ist damit verwendungsbereit.

### Beispiel 2

100.0 g Glycerooligolactid (GOL) der Zusammensetzung 1 : 26 (Teile Glycerin : Teile Milchsäure) werden in 150 ml N-Methylpyrrolidon gelöst. Zu 5 ml dieser Lösung werden 200 µg eines gepulverten Lyophilisats des basischen Fibrolasten-Wachstumsfaktors (bFGF), der, falls erwünscht, noch verschiedene Hilfsstoffe enthalten kann, gegeben. Die Mischung wird gleichmäßig verrührt und anschließend mit 25 ml eines porösen Granulats aus Knochenkeramik (Korngröße ca. 3 - 6 mm) vermischt. Das so erhaltene Produkt wird vor der Implantation noch 1 : 1 mit zerKleinerter Spongiosa vermischt und kann dann in einen Knochendefek implantiert werden.

Als gebrauchsfertiges Set besteht dieses aus folgenden Komponenten:
■ 5 ml Lösung aus GOL in NMP, ca 40 %ig
■ 200 µg bFGF-Lyophilisat (plus Hilfsstoffe, z.B. Sucrose)
■ 25 ml Knochenkeramikgranulat
■ Gefäße und Vorrichtungen zum Aufbewahren und Mischen
■ alle Komponenten sterilisiert.

## Patentansprüche

1. Poröses Implantatmaterial, dadurch gekennzeichnet, dass es mit einer Träger-Wirkstoff-Kombination beladen ist, wobei als Träger oligomere Ester aus mehrwertigen Alkoholen und α-Hydroxysäuren verwendet werden.

2. Implantatmaterial nach Anspruch 1, dadurch gekennzeichnet, dass als α-Hydroxysäuren Milchsäure, Glykolsäure, Caprolacton, Trimethylencarbonat oder α-Hydroxybuttersäure verwendet werden.

3. Implantatmaterial nach Anspruch 1 oder 2, dadurch gekennzeichnet, dass Mischungen von α-Hydroxysäuren untereinander und gegebenenfalls auch mit anderen Cooligomeren verwendet werden.

4. Implantatmaterial nach einem der Ansprüche 1 bis 3, dadurch gekennzeichnet, dass als Alkoholkomponente Glycerin, Ethylenglycol, Propylenglycol oder Polyethylenglycole verwendet werden.

5. Implantatmaterial nach einem der Ansprüche 1 bis 4, dadurch gekennzeichnet, dass als Implantatmaterial poröse Knochenersatzmaterialien, oberflächerstrukturierte Implantate, Vliese, Membranen, Gewebe oder auch Laminate verwendet werden.

6. Implantatmaterial nach einem der Ansprüche 1 bis 5, dadurch gekennzeichnet, dass als Wirkstoff pharmazeutische Wirkstoffe wie insbesondere Cytostatika, Antibiotika, Antiseptika, knochenwachstumsfördernde Substanzen, Wirkstoffe, die das Ein-wachsen von Gewebe in die poröse Strukur des Implantatmaterials beeinflussen oder gegebenenfalls auch geeignete Kombinationen mehrere dieser Wirkstoffe, enthalten sind.

7. Verfahren zur Herstellung eines Implantatmaterials nach einem der Ansprüche 1 bis 6, dadurch gekennzeichnet, dass man die oligomeren Ester in einem organischen Lösungsmittel auflöst und darin den oder die Wirkstoff(e) einbringt, und dass man anschließend diese Mischung auf das poröse Implantatmaterial aufbringt, das Lösungsmittel entfernt und gegebenenfalls das Implantatmaterial zuletzt steril verpackt.

8. Implantatmaterial nach einem der Ansprüche 1 bis 6, dadurch gekennzeichnet, dass es in Form eines gebrauchsfertigen Sets aus zwei oder mehr getrennten Komponenten vorliegt, dessen eine Komponente das poröse Implantatmaterial und eine andere Komponente eine Lösung der Träger-Wirkstoff-Kombination beinhaltet, oder gegebeneufalls auch die einzelnen Komponenten enthält, aus welchen die Träger-Wirkstoff-Kombination hergestellt werden kann.

9. Implantatmaterial nach Anspruch 8, dadurch gekennzeichnet, dass die Einzelkomponenten getrennt steril hergestellt und verpackt und bei Bedarf zum gebrauchsfertigen Implantatmaterial unter aseptischen Bedingungen kombiniert werden.
